# EUROPEAN PATENT APPLICATION

(11) **EP 4 268 887 A1**
(43) Date of publication of application: **01.11.2023**
(21) Application number: 22169961.4
(22) Date of filing: 26.04.2022
(51) Int. Cl.: A61N 5/10

(54) **A COMPUTER-BASED METHOD FOR EXTERNAL BEAM RADIATION TREATMENT PLANNING, A COMPUTER PROGRAM AND A COMPUTER FOR PERFORMING THE METHOD**

(71) Applicant: RaySearch Laboratories AB, 104 30 Stockholm (SE)
(72) Inventor: MARTHIN, Otte, 723 58 Västerås (SE); WASE, Viktor, 126 31 Hägersten (SE); FREDRIKSSON, Albin, 118 69 Stockholm (SE); ENGWALL, Erik, 129 44 Hägersten (SE); GLIMELIUS, Lars, 114 18 Stockholm (SE); BOKRANTZ, Rasmus, 182 47 Enebyberg (SE); ANDERSSON, Björn, 754 37 Uppsala (SE)

(57) **Abstract**

A computer-based method of optimizing a radiotherapy treatment plan for treating a patient involving radiation being delivered by a beam describing an arc is defined, the method including defining at least a first and a second part of the arc, the first part being a first sector of the arc and the second part being a second sector or a static beam, obtaining an optimization problem including at least a first optimization function for the first part, and a second optimization function for the second part and optimizing the radiotherapy treatment plan based on the optimization problem.

## Description

### Technical Field

The present invention relates to radiotherapy treatment planning and in particular to planning of radiotherapy treatment in which at least a portion of the treatment is delivered in the form of an arc.

### Background

External Beam Radiotherapy Treatment (EBRT) is a term that encompasses all types of radiotherapy treatment in which a radiation is delivered to a patient by means of a beam originating outside of the patient. The radiation can be in the form of photons or protons or other suitable types of ions, or electrons or other suitable subatomic particles. Several ways of obtaining such a plan are known in the art, including different optimization methods.

The process of optimizing a radiotherapy treatment plan, involves iteratively optimizing an optimization problem including a number of optimization functions, each representing a criterion that the resulting plan should fulfil. Two types of optimization functions are used: objective functions and constraints. An objective function sets a soft goal, specifying a result that the optimization should strive to get as close to as possible, for example, a target dose to a specific organ. A constraint is a hard limit that must not be violated, for example that a particular risk organ should receive less than a specified amount of radiation.

EBRT methods in which the radiation beam describes an arc over an area of the patient instead of one or more static beams are known for both photon and ion treatments. The arc is achieved by relative movement of the radiation source and the patient. Arc therapy is advantageous because it enables highly conformal dose distributions and, in many cases, faster delivery times.

It is an object of the present disclosure to provide a further improvement of arc radiotherapy treatment planning methods.

### Summary of the invention

The disclosure relates to a computer-based method of optimizing a radiotherapy treatment plan for treating a patient involving radiation being delivered by a beam describing an arc, the method including
- defining at least a first and a second part of the arc, the first part being a first sector of the arc and the second part being a second sector or a static beam,
- obtaining an optimization problem including at least a first optimization function related solely to the first part, and a second optimization function related to the second part,
- optimizing the radiotherapy treatment plan based on the optimization problem

Hence, the optimization method enables the control of various aspects of delivery, including the beam direction, or range of directions, from which a specific dose is to be delivered. This enables the calculation of more robust arc treatment plans than an arc that is not divided into sectors, because the dose delivered from each sector will be more homogeneous. If the patient moves in one direction, this may affect only the dose distribution from one part of the arc, while the dose distributions from the other part or parts may be unaffected. The target can be covered from all beam directions, or the target can be divided into sub-volumes. The dose to each sub-volume of the target can be delivered from the most suitable angle based on tissue homogeneity, and the proximity to organs at risk, which further increases robustness.

The resulting treatment plan includes two or more parts, where at least one part is always an arc, or an arc sector. The other parts may include any combination of sectors and static beams. Hence, the arc treatment plan may include one arc and one or more static beams, an arc divided into sectors, optionally with one or more static beams, or multiple arcs, similarly optionally divided into sectors and optionally one or more static beams. Plans including both an arc and a static beam are referred to in this document as "hybrid arcs". The simplest hybrid arc includes one arc and one static beam.

The second optimization function may be related solely to the second part, to a portion of the arc including the second part and one or more other parts, or to the whole arc. In other words, the optimization problem may include any suitable combination of optimization functions related to solely one of the parts of the arc, optimization functions related to one or more of the parts, and optimization functions related to the whole arc.

Several sectors and several static beams may be present. The order of delivery of the first, second, and any further parts is arbitrary, that is, the static beam can be delivered first, or last, or within one of the sectors or between two sectors.

In some preferred embodiments, the planning includes defining a first set of targets to receive radiation from the first part and a second set of targets, different from the first set of targets, to receive radiation from the second part. This further increases robustness, since the targets may be grouped according to the most suitable directions for delivering the radiation to each target with a view to distance to target, homogeneity or heterogeneity of the tissue traversed by the radiation, and/or the location of risk organs.

The method may comprise defining a first and a second sub-volume of a target that is to receive radiation and setting different dose objectives for the first sub-volume for the first and the second part of the arc. As an alternative, the method may comprise defining a first and a second sub-volume of a target that is to receive radiation and setting a first dose objective for the first sub-volume for the first part of the arc and a second dose objective for the second sub-volume for the second part of the arc. The first dose objective may be different than the second dose objective, or they may be the same. Both these alternatives enable the treatment of different sub-volumes in the target from different directions, depending on which direction is most suitable for each direction. Criteria such as distance to target, homogeneity or heterogeneity of the tissue traversed by the radiation, and/or the location of risk organs may be considered.

The method may further comprise defining a third part of the arc, the third part being a sector, and defining that no radiation should be delivered in the third part of the arc. In this way radiation can be minimized or completely avoided from directions which are unsuitable, for example, because of the location of a risk organ.

Hence, in some preferred embodiments, the planning involves selecting sectors in which radiation should be delivered or not delivered, respectively, to the patient. If the plan is a Pencil Beam Scanning (PBS) plan used in proton and ion therapy, such preferred embodiments may include selecting spot positions for each sector. If the plan is a photon-based plan, such preferred embodiments may include defining sectors in which non-zero fluence is allowed, or where the Multi-Leaf Collimator (MLC) and/or jaws are allowed to be open.

The arc may define a full revolution around the patient or may extend over a portion of the patient's circumference. The arc may move over the same trajectory only once, or two or more times. In the latter case, the sectors are preferably the same each time, but they may also be different. In the case where the sectors are the same for multiple traversals, the selection of sectors where the radiation should be delivered, as well as the selection of optimization functions could be the same for all traversals or be set individually per traversal. The arc may describe several revolutions around the patient or may move back and forth over the same trajectory, or may move according to any other suitable pattern, for example a zig-zag pattern to cover a larger area of the patient.

In another aspect, the disclosure relates to a method of optimizing a radiotherapy treatment plan for treating a patient involving radiation being delivered by a beam describing an arc, the method including
- defining at least a first and a second part of the arc, the first part being a first sector of the arc and the second part being a second sector or a static beam,
- defining a first set of targets to receive radiation from the first part and a second set of targets, different from the first set of targets, to receive radiation from the second part
This may be used for certain applications, if it is important to steer from which direction the radiation is delivered to the target, or to each target, or to each part of a target, but not necessarily to vary the dose level from each direction.

The disclosure also relates to a computer program product comprising computer-readable code means which, when run in a computer, will cause the computer to perform any of the methods as disclosed above. The computer program product may include a non-transitory storage medium on which the code means are stored. The disclosure also relates to a computer comprising a processor and a program memory said program memory comprising such a computer program product so that it can be executed in the processor.

### Brief description of drawings

The invention will be described in more detail in the following, by way of examples and with reference to the appended drawings.
Fig. 1 illustrates a patient and arc sectors in an example involving photon treatment planning.
Fig. 2 illustrates a patient and arc sectors in an example involving proton treatment planning
Fig. 3 is a flow chart of a first method according to the present disclosure.
Fig. 4 is a flow chart of a second method according to the present disclosure.
Fig. 5 is a schematic illustration of a computer system in which the methods of this disclosure may be carried out.

### Detailed description of embodiments

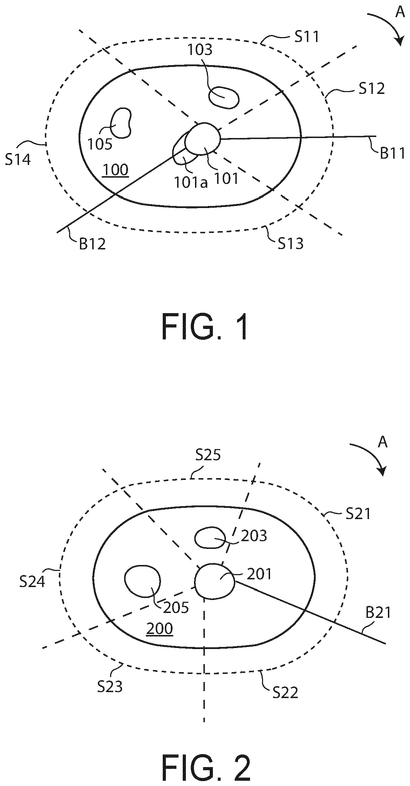

Fig. 1 illustrates, by way of example only, aspects that may be considered when planning a photon-based treatment for a patient. Fig. 1 shows a cross-section through a patient 100 with a target 101 and an organ at risk 103. There is also an area 105 having significantly different properties from the surrounding tissue, such as bone or an air-filled cavity causing inhomogeneity. A treatment plan has been developed for the patient, including an arc shown as a dashed elliptic form surrounding the patient 100, which has been divided into a first sector S11, a second sector S12, a third sector S13 and a fourth sector S14. The arc is created by relative movement between the radiation source and the patient, typically by rotation of the gantry or the patient support (not shown) The direction of the arc in this example is clockwise, as indicated by an arrow A. Within the second sector S12 there is a first static beam B11, and between the second S12 and third S13 sectors there is a second static beam B12.

The beginning and end of the first sector S11 are marked with dashed lines. As can be seen, within the first sector, the organ at risk 103 is located between the radiation source (not shown) and the target 101. Therefore, the plan has been devised to provide no or a small portion of radiation in this sector S11. Providing no radiation may be achieved by setting the fluence to zero or by closing the collimator leaves or jaws. Providing a smaller portion of radiation can be achieved by defining an optimization function with a lower dose objective, which will promote the optimization to adjust the variables in order to reduce the radiation and thus reduce the dose from this direction. For the second sector, in this very simplified example, there is no organ at risk between the radiation source and the target, and also no organ at risk on the other side of the target in the direction of the beam. The optimization function for the second sector, therefore, could say that a certain percentage of the dose should be delivered from the second sector. Similarly, the optimization function for the first static beam B11 located within the second sector, S12, could say that a major fraction of the dose should be delivered by this static beam, since it is positioned so that it can avoid the organ at risk 103. The optimization functions could include other quantities important for the outcome of the treatment, such as number of monitor units, or delivery time, or linear energy transfer in proton and ion treatments, or biological measures based on normal tissue complication probability and tumor control probability.

The second static beam B12, located between the third S13, and fourth S14 sectors, is well positioned for treating one particular sub-volume 101a of the target 101. Accordingly, an optimization function for this static beam could say that it should provide all the dose to this particular sub-volume 101a. In the third sector, the beam will intersect with the organ at risk 103 after passing the target, which means that any beam from this angle would result in dose to the organ at risk. Therefore, the optimization problem is set to provide no radiation in this sector S 13, or to say that only a minor fraction of the dose should be delivered in this sector. In the fourth sector, the inhomogeneity means that passing through this structure may result in less robust plans, since small changes in the patient anatomy or setup could result in large differences in the dose distribution. Alternatively, the density of the structure may be such that it is unknown how it affects the dose distribution, for example in the case of a titanium implant. In such cases, the dose behind the structure from a beam traversing the structure may be uncertain even if there are no changes to the patient anatomy or setup. For these reasons, it can be desirable to limit the radiation from this sector in order to increase overall robustness.

In addition to defining arc parts, that is, sectors and static beams and specifying dose objectives for each part, one or more sets of targets, or target sub-volumes may be defined and each arc part may be assigned one set of targets that will receive dose from that part. This involves determining whether non-zero fluence is allowed in a respective part, and where MLC and/or jaws are allowed to be open.

Fig. 2 illustrates, by way of example only, aspects that may be considered when planning a proton-based treatment for a patient. Fig. 2, like Fig. 1, shows a cross-section through a patient 200 with a target 201 and an organ at risk 203. There is also an area 205 having significantly different properties from the surrounding tissue, such as bone or an air-filled cavity causing inhomogeneity. A treatment plan has been developed for the patient, including an arc, shown as a dashed elliptic form which has been divided into a first, a second, a third, a fourth and a fifth sector S21, S22, S23, S24, S25, respectively. As for Fig. 1, the arc in this example is created by relative movement between the radiation source and the patient and the direction of the arc is clockwise, as indicated by an arrow A. Between the first S21 and the second sector S22 there is a first static beam B21.

For the first and third sectors, S21, S23, there is little risk of hitting the risk organ 203. Therefore, the sector specific optimization functions could specify that a certain fraction of the dose should be delivered in each of these sectors. For the second sector S22 the fraction of the dose to be delivered may be set lower, because of the proximity to the risk organ 203, which will be located behind the target 201 seen in the direction of the beam. Alternatively, only the parts of the target located at a certain distance from the risk organ will be treated from this direction, in order to ensure that no dose will be given to the risk organ from this direction. For the fourth sector S24, the inhomogeneity 205 can be considered in the optimization. Passing through this structure may result in less robust plans, since small changes in the patient anatomy or setup could result in large differences in the range of the protons and thus in the dose distribution. Alternatively, the density of the structure may be such that it is unknown how it affects the dose distribution, for example in the case of a titanium implant. In such cases, the dose behind the structure from a beam traversing the structure may be uncertain even if there are no changes to the patient anatomy or setup. For these reasons, it can be desirable to limit the radiation from this sector in order to increase overall robustness. This is even more important for ions such as protons than for photons, since ions are much more sensitive to different uncertainties that can occur during treatment.

For the fifth sector, S25, the organ at risk 203 is located between the source of the radiation and the target 201. Therefore, it may be determined that no dose should be delivered in the fifth sector S25, that is, the beam should be turned off in this sector.

In addition to defining arc parts, that is, sectors and static beams and specifying dose objectives for each part, one or more sets of targets, or target sub-volumes may be defined and each arc part may be assigned one set of targets that will receive dose from that part. This involves determining spot placement for each part of the beam.

In the examples shown in Figs. 1 and 2, it is assumed that the arc extends all the way around the patient once, which is not necessarily the case. For both ion and photon treatments, the arc could also extend around just a part of the patient's circumference, possibly moving back and forth over that part. The arc could also include more than one full revolution around the whole patient. Also, the arc does not have to describe a smooth curve but could move across the patient in any suitable pattern. There could be just a first and a second sector, or a first sector and a first static beam, or any suitable combination of sectors and static beams. In both examples, a static beam may be located at the beginning or end of a sector or within a sector. The sectors may all have the same length, or different length sectors may be defined as in Figs. 1 and 2.

If the arc extends more than one revolution around the patient, it is usually preferable to set up the optimization functions in such a way that the sectors remain the same and are treated in the same way, for all revolutions. This is also the case for other situations where the beam coves the same area more than once, for example, by a back-and-forth movement. The optimization problem may alternatively be set up with different optimization functions for each time the beam passes over a particular area.

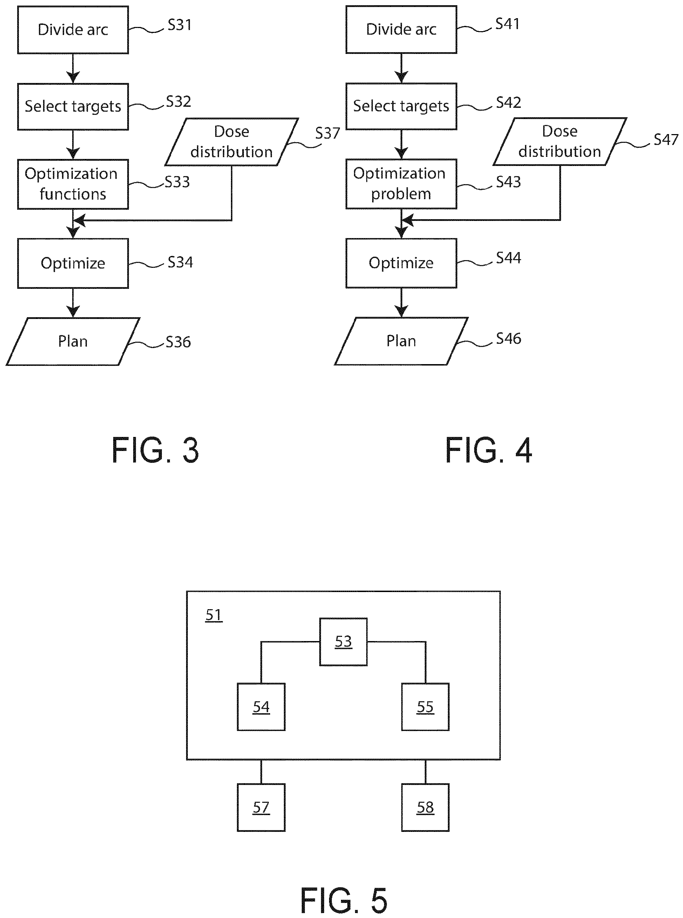

Fig. 3 is a flow chart of an embodiment of a method of optimizing a radiotherapy treatment plan. In a first step S31 an arc is defined and divided into parts, including at least one sector and at least one second part which is either a sector or a static beam. In an optional second step S32, one or more targets are selected to be covered by each sector. This may involve dividing the target into different parts and letting different sectors of the arc cover the different parts of the target. It may also be specified that a particular sector should avoid one or more targets. Step S32 may be performed manually, or automatically, for example based on tissue homogeneity and/or the presence or absence of any organ at risk in the trajectory towards the target, or part or the target. For ion treatment plans, such as proton treatment plans, this may involve determining spot placement. For photon treatment plans it will typically involve defining areas where non-zero fluence is allowed, and/or where MLC and/or jaws are allowed to be open.

In a subsequent step S33, an optimization problem is defined, with a number of optimization functions. As explained above, optimization functions can be soft objective functions or hard constraints. In the present method, each part of the arc has its own set of optimization functions. Also, normally some optimization functions will be common for the whole arc. There may also be optimization functions that are valid for a subset of the parts, for example, for two or more adjacent sectors, for two opposite sectors, for a static beam and two adjacent sectors on either side of the static beam, or for one sector and a static beam located within the sector. In step S34, optimization is performed using the optimization problem. In some cases additional input data, such as a reference dose distribution S37, may also be used. The output S36 is an optimized plan comprising an arc with the defined sectors and static beams according to step S31.

Fig. 4 illustrates an alternative method without different optimization functions for the different sectors or static beams. In a first step S41 an arc is defined and divided into parts, including at least one sector and at least one second part which is either a sector or a static beam. In a second step S42, for each sector, one or more targets are selected to be covered by each part of the arc. This may involve dividing the target into different parts and letting different parts of the arc cover the different parts of the target. In other words, one target may be covered by a static beam while another target is covered by one or more sectors. In the general case, two or more different subsets of targets may be defined, and each subset may be assigned to one or more parts of the arc. For pencil beam scanning methods, this involves spot placement. For photon-based methods it involves controlling fluence, MLC leaf positions and/or jaws.

In a subsequent step S43, an optimization problem is defined for the full set of targets. In step S44, optimization is performed using the optimization problem. In some cases, additional input data, such as a reference dose distribution S47, may also be used. For pencil beam scanning methods, this involves spot placement. For photon-based methods it involves controlling fluence, MLC leaf positions and/or jaws. The output S46 is an optimized plan comprising an arc with the defined sectors and static beams according to step S41.

Fig. 5 illustrates schematically a computer system in which one or both methods of the present disclosure may be performed. A computer 51 comprises a processor 53, a data memory 54 and a program memory 55. Preferably, one or more user input means 57, 58 are also present, in the form of a keyboard, a mouse, a joystick, voice recognition means or any other available user input means. The user input means may also be arranged to receive data from an external memory unit.

The data memory 54 comprises input data to be used in the procedures, such as patient images, dose objectives, information about sectors and/or target parts. The data memory may also comprise output data from the procedures. The treatment plan may be generated in the computer 51, or received from another storage means in any way known in the art.

As will be understood, the data memory 54 is only shown schematically. There may be several data memory units, each holding one or more different types of data, for example, one data memory for the treatment plan, one for the patient images, etc. The program memory 55, also only shown schematically, holds a computer program arranged to control the processor to perform one or more of the methods disclosed in this application.

## Claims

1. A computer-based method of optimizing a radiotherapy treatment plan for treating a patient involving radiation being delivered by a beam describing an arc, the method including
• defining at least a first and a second part of the arc, the first part being a first sector of the arc and the second part being a second sector or a static beam,
• obtaining an optimization problem including at least a first optimization function related solely to the first part, and a second optimization function related to the second part,
• optimizing the radiotherapy treatment plan based on the optimization problem.

2. A method according to claim 1, wherein the second optimization function is related solely to the second part.

3. A method according to claim 1, wherein the second optimization function is related to the whole arc.

4. A method according to claim 1, further comprising defining a first set of targets to receive radiation from the first part and a second set of targets, different from the first set of targets, to receive radiation from the second part.

5. A method according to claim 1, further comprising defining a first and a second sub-volume of a target that is to receive radiation and setting different dose objectives for the first sub-volume for the first and the second part of the arc.

6. A method according to claim 1, further comprising defining a first and a second sub-volume of a target that is to receive radiation and setting a first dose objective for the first sub-volume for the first part of the arc and a second dose objective for the second sub-volume for the second part of the arc, the first dose objective being different than the second dose objective.

7. A method according to any one of the preceding claims, further comprising defining a third part of the arc, the third part being a sector, and defining that no radiation should be delivered in the third part of the arc.

8. A method according to any one of the preceding claims, wherein the arc defines a full revolution around the patient.

9. A method according to any one of the claims 1-7, wherein the arc extends over a portion of the patient's circumference.

10. A method according to any one of the preceding claims, wherein the arc moves over the same trajectory two or more times, and the sectors are the same each time.

11. A computer-based method of optimizing a radiotherapy treatment plan for treating a patient involving radiation being delivered by a beam describing an arc, the method including
• defining at least a first and a second part of the arc, the first part being a first sector of the arc and the second part being a second sector or a static beam,
• defining a first set of targets to receive radiation from the first part and a second set of targets, different from the first set of targets, to receive radiation from the second part

12. A computer program product comprising computer-readable code means which, when run in a computer, will cause the computer to perform the method according to any one of the preceding claims.

13. A computer program product according to claim 12, comprising non-transitory storage medium whereon the computer readable code means are stored.

14. A computer (51) comprising a processor (53) and a program memory (55) said program memory comprising a computer program product according to claim 12.
